# EUROPEAN PATENT APPLICATION

(11) **EP 4 488 368 A1**
(43) Date of publication of application: **08.01.2025**
(21) Application number: 23184209.7
(22) Date of filing: 07.07.2023
(51) Int. Cl.: C12N 9/02

(54) **NAD(P)H OXIDASE**

(71) Applicant: Annikki GmbH, 8074 Raaba-Grambach (AT)
(72) Inventor: Staunig, Nicole, 8074 Raaba-Grambach (AT); Dupont, Maria, 8074 Raaba-Grambach (AT); Koch, Dennis, 8074 Raaba-Grambach (AT)
(74) Representative: Schwarz & Partner Patentanwälte GmbH

(57) **Abstract**

The present invention relates to a protein having NADH and/or NADPH oxidase activity comprising an amino acid sequence selected from the group consisting of
i) an amino acid sequence having at least 80% sequence identity with SEQ ID No. 1,
ii) an amino acid sequence encoded by a nucleic acid sequence having an identity to SEQ ID No. 2 of at least 80%, and
iii) an amino acid sequence encoded by a nucleic acid which binds under stringent conditions to a nucleic acid molecule complementary to the nucleic acid sequence SEQ ID No. 2, wherein the stringent conditions preferably comprise washing at 65°C, and at a salt concentration of 0.1 to 2x SSC.

## Description

### TECHNICAL FIELD

The present invention relates to the field of enzymes, in particular enzymes capable to oxidase NADH and/or NADPH.

### BACKGROUND ART

Biocatalytic redox processes are widely used to replace more expensive chemical synthesis, as no extensive amounts of waste are formed, and the processes can be performed in shorter time using milder reaction conditions (ambient pressure and temperature) .

Redox reactions like the stereoselective reduction of keto groups or the regioselective oxidation of hydroxy groups are of special interest for pharma and food industries and are mostly performed by oxidoreductases, accompanied by the interconversion of the nicotinamide cofactors NAD(P)⁺ (oxidized form) and NAD(P)H (reduced form). These redox reactions require a stoichiometric amount of NAD(P)⁺ or NAD(P)H for the conversion to be completed, however, the addition of such cofactors in industrial processes is very expensive and not cost-efficient.

Introducing an additional enzyme to the reaction mixture, which converts the used cofactor back to the oxidation state (NAD(P)⁺ or NAD(P)H) necessary for the oxidoreductase-catalyzed redox reaction (either by oxidation or reduction), can greatly reduce the amount of expensive nicotinamide cofactor. This method is known as *cofactor regeneration* and is widely used in industrial enzymatic processes (Chenault et al., 1988; Wang et al., 2017).

There are enzymatic methods known in the art for regeneration of the reduced nicotinamide cofactor NAD(P)H - e.g., glucose and glucose dehydrogenase (GDH), formate and formate dehydrogenase (FDH) or a secondary alcohol (like isopropanol) and alcohol dehydrogenase (ADH). For the regeneration of the oxidized nicotinamide cofactor NAD(P)⁺ systems like pyruvate and lactate dehydrogenase (only for NAD⁺ regeneration), acetaldehyde and alcohol dehydrogenase or α-ketoglutarate and glutamate dehydrogenase can be employed (Chenault et al., 1988).

All these methods require the addition of a second substrate to the reaction mixture, which makes it more expensive, because further processing steps (such as removal of the coproducts or reutilization of the substrate used for cofactor regeneration, if possible) are necessary. In complex reaction cascades with multiple biocatalytic steps each additional (co)substrate can complicate the downstream processing, serve as alternative substrate for the cascade enzymes, act as a noncompetitive inhibitor or denature the proteins.

NAD(P)H oxidases, a type of oxidoreductases, allow the oxidation of NAD(P)H to NAD(P)⁺ without concomitant use of an additional substrate - oxygen is used as substrate while only generating water or hydrogen peroxide (water- or hydrogen peroxide-forming NAD(P)H oxidases, respectively).

As the oxidizing compound hydrogen peroxide can denature or degrade enzymes, water-forming NAD(P)H oxidases are preferred in industrial applications. Examples for such enzymes are the NADH oxidase from *Lactobacillus brevis* (Geueke et al, 2003; EP 1 285 962 A1; US 7,435,574 B2), *Pyrococcus furiosus* (Ward et al., 2001), *Borrelia burgdorferi* (Riebel et al., 2002) and *Streptococcus mutans* (Higuchi et al., 1993; Matsumoto et al., 1996; Higuchi et al., 1999), as well as the NAD(P)H oxidase from *Lactobacillus sanfranciscensis* (Riebel et al., 2003). The drawback of these enzymes is their low total turnover numbers (TTN) due to the instability in oxidative process conditions.

Recently, engineered versions of the NADH oxidase from *Streptococcus mutans* were described, which are more stable showing no loss of activity and thus can be employed for cofactor regeneration in redox processes without requiring the use of antioxidant agents like dithiothreitol (EP 2 527 436 B1; US 9,315,782 B2; US 9,376, 667 B2). However, the prior art does not reveal the performance of the NADH oxidase from *Streptococcus mutans* under process conditions, like in presence of organic solvents which have denaturing effects (Klibanov, 1997). Thus, there is still a need for alternative enzymes for cofactor regeneration in oxidative process conditions.

One important class of pharmaceuticals are bile acids and their derivatives. Cholic acid, e.g. can be used as starting material for the chemical synthesis of ursodeoxycholic acid (for treatment of gallstones) involving two main steps - the dehydroxylation at C12 and the epimerization of the hydroxy group at C7. For most of the reactions enzymatic alternatives using highly specific hydroxysteroid dehydrogenases are described literature, only the reduction of the ketone moiety at C12 to a methylene group has to be done chemically via a Wolff-Kishner reduction (Tonin and Arends, 2018). For the oxidation reactions required in both main steps NAD(P)H oxidases can be used for cofactor regeneration (Tonin et al., 2019).

Bile acids like cholic acid or deoxycholic acid are known as protein unfolding and protein aggregation-inducing agents in bacteria (Cremers et al., 2014), which introduces a further obstacle to industrial enzymatic processes, where high concentrations of bile acid substrate are indispensable for cost-effective production of chemicals.

Another application of NAD(P)H oxidases as cofactor regenerating enzymes is the oxidation of sugar acids to the corresponding aldoses or ketoses. Of special interest are so-called rare sugars, which are not abundant in nature. One of these sugars is D-psicose or D-allulose, which can be used as low-calorie sweetener. D-psicose can be produced by the enzymatic epimerization of D-fructose, however, due the unfavorable chemical equilibrium of the epimerization, only conversions of max. 38% can be achieved (Zhang et al., 2016; Jiang et al., 2020). An elegant approach to solving this problem was presented by Wang et al. The authors used an *Escherichia coli* whole cell catalyst harboring an epimerase and a ribitol dehydrogenase to convert the D-fructose to the sugar alcohol allitol (via D-psicose as intermediate). Allitol was then re-oxidized to D-psicose employing a second *E. coli* whole cell catalyst harboring another ribitol dehydrogenase and an NADH oxidase from *Streptococcus pyogenes.* 90% of the D-fructose could be converted to D-psicose (Wang et al., 2023).

These examples show only a small part of the broad substrate scope of enzymatic oxidations involving NAD(P)H oxidases for the regeneration of NAD(P)⁺.

One object of the present invention is to provide an NAD(P)H oxidase, which shows activity in presence of different classes of organic substrates (like bile acids or sugars/sugar alcohols) and is characterized by a high stability towards organic solvents, especially useful for one-pot or cascade reactions, without the use of expensive additives for the stabilization of the biocatalyst.

### SUMMARY OF THE INVENTION

Hence, the present invention relates to a protein, preferably stable protein, having NADH and/or NADPH oxidase activity comprising an amino acid sequence selected from the group consisting of
i) an amino acid sequence having at least 80% sequence identity with SEQ ID No. 1,
ii) an amino acid sequence encoded by a nucleic acid sequence having an identity to SEQ ID No. 2 of at least 80%, and
iii) an amino acid sequence encoded by a nucleic acid which binds under stringent conditions to a nucleic acid molecule complementary to the nucleic acid sequence SEQ ID No. 2, wherein the stringent conditions preferably comprise washing at 65°C, and at a salt concentration of 0.1 to 2x SSC.

Surprisingly, it turned out that a protein as defined above exhibiting an NADH and/or NADPH oxidase activity shows a significant higher stability compared to other known NADH and/or NADPH oxidases. The activity of the protein of the present invention remains stable for a much longer period of time compared to other NADH and/or NADPH oxidases. This allows to increase the yield of NAD⁺ and/or NADP⁺, which is important if the protein of the present invention is used in a cofactor recycling system or as a cofactor recycling system, for instance. Also the presence of organic co-solvents has a much lower effect on the enzymatic activity of the protein of the present invention compared to other NADH and/or NADPH oxidases. Hence, the protein of the present invention can be considered as a stable or organic solvent stable enzyme, i.e. NADH and/or NADPH oxidase.

It was also surprising that the enzymatic activity of the protein of the present invention is less influenced by the presence of a plurality of other compounds and proteins compared to other NADH and/or NADPH oxidases. This property is particularly advantageous when the protein of the present invention is used in multi-enzymatic reactions involving several other enzymes and compounds for regenerating NAD⁺ and/or NADP⁺ (i.e. cofactor recycling) which are required by oxidoreductases, for instance.

Due to the fact that the protein of the present invention is able to oxidize NADH and/or NADPH, said protein can also be considered as a NAD(P)H, NADH and/or NADPH oxidase.
SEQ ID No. 1: 
MKVVVVGCTHAGTAAVKTILNEHPDASVSVYERNDNVSFLSCGIALYVGGVVKDPAGLFYSS
PEELASMGAKINMEHNVKNIDNENKVVVIENLKTGETFEESYDKLVMTTGSWPIIPPIDGIN
SENILLCKNYNQANEIIKESKNAKKIVIVGGGYIGIELVEAFAESGKQVTLVDGLDRILNKY
LDAEFTSVLEHDLQERGVTLALNQTVEKFVANESGAVTAVKTPVGEYEADLVILCVGFKPNT
DLLKDKVEMLPNGAIVVDEYMRTSDEAIFAAGDSCAVHYNPTGGSAYIPLATNAVRMGALVG
KNIVSPTVKYRGTQATSGLYLFGFNIGSTGLTENSAPHFGVEVRSVVVEDNYRPEFMPTTEK
VTMKLVYEVGTNRIVGGQIMSKYDVTQSANTLSLCVQNKMTIEDLAYVDFFFQPHFDRPWNY
LNILAQAAVEQERKLAK
SEQ ID No. 2:

ATGAAAGTAGTAGTAGTAGGCTGTACACATGCAGGAACAGCGGCAGTTAAGACG
ATTTTAAATGAACATCCAGATGCATCAGTATCAGTATATGAGCGTAATGACAATGTCTCA
TTTCTATCTTGTGGGATTGCGTTGTATGTTGGTGGAGTTGTGAAAGATCCTGCAGGTTTG
TTTTATTCAAGTCCAGAAGAACTTGCATCAATGGGCGCGAAAATTAACATGGAACACAAT
GTGAAAAATATAGATAATGAGAATAAGGTCGTAGTAATTGAGAATTTAAAAACAGGCGAA
ACATTTGAAGAAAGCTATGATAAGTTGGTAATGACAACTGGATCATGGCCAATTATTCCT
CCAATTGATGGAATCAATAGTGAAAATATTCTTTTGTGTAAAAACTATAACCAAGCAAAT
GAAATTATTAAAGAATCAAAAAATGCTAAAAAGATTGTCATTGTTGGTGGTGGCTATATT
GGAATTGAATTAGTTGAGGCATTTGCAGAATCTGGCAAGCAAGTGACGCTAGTTGATGGA
TTAGATCGTATTTTAAACAAATATTTAGATGCTGAATTCACTTCTGTTTTAGAGCATGAT
TTACAAGAAAGAGGCGTTACGCTAGCTTTAAACCAAACCGTCGAGAAATTTGTTGCCAAT
GAATCAGGTGCTGTGACAGCTGTGAAAACACCAGTTGGAGAATATGAGGCTGATTTAGTT
ATTTTATGTGTTGGATTTAAACCAAATACTGATTTGTTGAAGGATAAAGTAGAGATGTTG
CCAAATGGTGCCATCGTAGTGGATGAATATATGAGAACAAGCGATGAAGCGATTTTTGCT
GCTGGCGATAGTTGCGCGGTTCATTATAATCCAACTGGAGGCTCTGCGTATATTCCGTTA

GCTACAAATGCAGTTAGAATGGGAGCTTTAGTTGGGAAAAATATTGTTTCTCCAACAGTT
AAATATCGTGGCACGCAAGCAACTTCTGGTTTATATTTATTTGGTTTTAATATAGGTTCA
ACCGGATTGACTGAAAATAGCGCTCCTCATTTTGGCGTAGAGGTTCGTTCAGTAGTTGTA
GAAGATAATTATCGTCCAGAGTTTATGCCGACAACAGAGAAAGTAACGATGAAATTAGTT
TATGAAGTAGGAACGAATCGGATTGTTGGAGGTCAAATCATGTCAAAATATGATGTGACA
CAATCTGCCAATACGTTATCTTTATGTGTTCAAAATAAAATGACGATTGAGGATTTGGCT
TATGTAGATTTCTTCTTCCAACCTCACTTTGATCGTCCTTGGAACTATTTAAATATTTTA
GCGCAAGCAGCTGTTGAGCAAGAGCGTAAACTAGCAAAATAA

The protein of the present invention comprising or consisting of SEQ ID No. 1 is a new NADH oxidase isolated from gram-positive bacteria of the genus *Carnobacteria,* e.g. *Carnobacterium divergens,* and can be used, for instance, in industrial processes for cofactor regeneration. This NADH oxidase as well as the proteins of the present invention are characterized by high total turnover numbers (TTN) and are stable even in processes, where critical substances, which can lead to denaturation, degradation and/or inhibition of the enzyme, are converted. The protein of the present invention is also able to act as NADPH oxidase or even as NAD(P)H oxidase as described in more detail below.

A further aspect of the present invention relates to a nucleic acid molecule encoding a protein according to the present invention, as defined above.

The nucleic acid molecule of the present invention can be part of a vector, for instance. Hence, a further aspect of the present invention relates to a vector comprising a nucleic acid molecule according to the present invention.

A further aspect of the present invention relates to a host cell comprising a nucleic acid molecule or a vector according to the present invention.

The nucleic acid molecule and/or the vector of the present invention can be part of a host cell. It is particularly preferred that the host cell of the present invention is able to express a protein of the present invention from said nucleic acid molecule and/or said vector. The protein can be secreted from the host cell or expressed intracellularly.

Another aspect of the present invention relates to the use of a protein according to present invention or a host cell according to the present invention or a lysate or homogenate thereof for oxidizing NADH to NAD⁺ and/or NADPH to NADP⁺.

The protein of the present invention can be used to oxidize NADH to NAD+ and/or NADPH to NADP⁺. A lysate or a homogenate from a host cell expressing the protein of the present invention can be used to oxidize NADH to NAD+ and/or NADPH to NADP⁺ as well.

A further aspect of the present invention relates to a method for oxidizing NADH to NAD⁺ and/or NADPH to NADP⁺ comprising the step of incubating a protein or a lysate of a host cell according to the present invention with NADH and/or NADPH.

Another aspect of the present invention relates to a protein or a host cell according to the present invention or a lysate or a homogenate thereof in a cofactor recycling system or as a cofactor recycling system.

An aspect of the present invention relates to a method for the enzymatic oxidation of a compound, where NADH is formed from NAD⁺ and/or NADPH is formed from NADP⁺, comprising the step of oxidizing NADH to NAD⁺ and/or NADPH to NADP⁺ by adding a protein or a lysate of a host cell according to the present invention.

### SHORT DESCRIPTION OF THE FIGURES

Fig. 1 shows a comparison of enzymatic stability between *Streptococcus mutans* NOX (SmNOX, grey lines) and *Carnobacterium divergens* NOX (CdNOX, black lines) in presence of isopropanol (IPA) (A) and acetone (B).
Fig. 2 shows the chemical structures and conversions of examples 6 to 8.

### DESCRIPTION OF EMBODIMENTS

Amino acid classification, as used herein, is as follows:
Polar amino acids are Serine (Ser, S), Threonine (Thr, T), Cysteine (Cys, C), Glutamine (Gln, Q) and Asparagine (Asn, N). Positively charged amino acids are Lysine (Lys, K) and Arginine (Arg, R) as well as Histidine (His, H). Negatively charged amino acids are Aspartate (Asp, D) and Glutamate (Glu, E). Non-polar amino acids are Glycine (Gly, G), Alanine (Ala, A), Valine (Val, V), Leucine (Leu, L), Isoleucine (Ile, I), Methionine (Met, M), Proline (Pro, P), Phenylalanine (Phe, F), Tyrosine (Tyr, Y) and Tryptophan (Trp, W).

The terms "sequence identity" and "identity", as used herein, refer to the percentage of identical nucleotide or amino acid matches between at least two nucleotide or amino acid sequences aligned using a standardized algorithm. Such an algorithm can, in a standardized and reproducible manner, insert gaps in the compared sequences to optimize the alignment between two sequences, thus achieving a more meaningful comparison of the two sequences.

Percent identity between sequences may be determined using one or more computer algorithms or programs known in the prior art or described herein. According to the present invention, the Basic Local Alignment Search Tool (BLAST) provided by the National Center for Biotechnology Information (NCBI) (Altschul et al., 1990), is preferably used. The BLAST software series contains several programs, including a tool referred to as "BLAST 2 Sequences", which is used for direct pairwise comparison of two nucleotide or amino acid sequences. "BLAST 2 Sequences" can also be accessed and used interactively via the NCBI World Wide Web page on the Internet. The blastn program (for nucleotide sequences) uses as defaults a word length (W) of 28, an expectation (E) of 0.05, M = 1, N = -2, and a comparison of both strands. For amino acid sequences, the blastp program uses as defaults a word length of 3 and an expectation (E) of 0.05 and the BLOSUM62 scoring matrix (Henikoff & Henikoff, 1989), alignments (B) of 50, expectation (E) of 0.05, M = 1, N = -2.

Alternatively, the NADH and/or NADPH oxidase of the present invention preferably comprises an amino acid sequence encoded by a nucleic acid that binds under stringent conditions to a nucleic acid molecule complementary to the nucleic acid sequence SEQ ID No. 2. As used herein, stringent conditions refer to conditions under which so-called specific hybrids, but not non-specific hybrids, are formed.

Hybridization may be performed by conventionally known procedures, such as those described in J. Sambrook et al, Molecular Cloning, A Laboratory Manual, 2nd Ed, Cold Spring Harbor Laboratory (1989). The stringent condition is a condition in which washing is performed at 65°C, and at a salt concentration of 0.1 to 2x SSC (wherein 1xSSC is understood to be a mixture of 0,15 M sodium chloride/0,015 M sodium citrate).

According to a preferred embodiment of the present invention the protein of the present invention comprises a polypeptide consisting of the amino acid sequence GX₁GX₂X₃X₄, wherein X₁ is a non-polar amino acid residue, X₂ is a non-polar amino acid residue, X₃ is a non-polar amino acid residue and X₄ is a non-polar amino acid residue.

According to another preferred embodiment of the present invention X₁ is glycine, and/or X₂ is tyrosine, and/or X₃ is isoleucine, and/or X₄ is glycine or alanine. Hence, according to a particularly preferred embodiment the protein of the present invention comprises the amino acid sequence GGGYIX₄ (SEQ ID No. 3), even more preferably GGGYIG (SEQ ID No. 4) or GGGYIA (SEQ ID No.5). Surprisingly, it turned out that a protein of the present invention comprising the amino acid sequence GGGYIG (SEQ ID No. 4) is able to oxidize NADH and/or NADPH, whereas a protein of the present invention comprising the amino acid sequence GGGYIA (SEQ ID No. 5) shows a significantly reduced or even no capability to oxidize NADH. The latter proteins are thus used preferably to oxidize NADPH.

According to a preferred embodiment of the present invention the protein of the present invention comprises a polypeptide consisting of the amino acid sequence GX₁GX₂X₃X₄X₅X₆X₇X₈X₉X₁₀X₁₁X₁₂, preferably GGGYIX₄X₅X₆X₇X₈X₉X₁₀X₁₁X₁₂ (SEQ ID No. 6), GGGYIGX₅X₆X₇X₈X₉X₁₀X₁₁X₁₂ (SEQ ID No. 7) or GGGYIAX₅X₆X₇X₈X₉X₁₀X₁₁X₁₂ (SEQ ID No. 8), wherein X₁ is a non-polar amino acid residue, X₂ is a non-polar amino acid residue, X₃ is a non-polar amino acid residue, X₄ is a non-polar amino acid residue, X₅ is a peptide consisting of 6 to 12, preferably 8 to 10, more preferably 10, amino acid residues, X₆ is a polar amino acid residue, X₇ is a peptide consisting of 4 to 8, preferably 4 to 6, more preferably 6, amino acid residues, X₈ is an acidic amino acid residue or a non-polar amino acid residue, X₉ is a polar amino acid residue or a basic amino acid residue, X₁₀ is a non-polar amino acid residue or a polar amino acid residue, X₁₁ is a peptide consisting of 2 to 6, preferably 2 to 4, more preferably 4, amino acid residues, and X₁₂ is a polar amino acid residue or a basic amino acid residue, wherein X₁ is particularly preferred glycine, and/or X₂ is particularly preferred tyrosine, and/or X₃ is particularly preferred isoleucine, and/or X₄ is particularly preferred glycine or alanine.

According to another preferred embodiment of the present invention X₅ is a peptide consisting of amino acid sequence IELVEAFAES (SEQ ID No. 9) and/or X₇ is a peptide consisting of amino acid sequence KQVTLV (SEQ ID No. 10) and/or X₁₁ is a peptide consisting of amino acid sequence DRIL (SEQ ID No. 11).

The protein of the present invention preferably comprises a polypeptide consisting of amino acid sequence GX₁GX₂X₃X₄IELVEAFAESX₆KQVTLVX₈X₉X₁₀DRILX₁₂ (SEQ ID No. 12), more preferably GGGYIX₄IELVEAFAESX₆KQVTLVX₈X₉X₁₀DRILX₁₂ (SEQ ID No. 13), GGGYIGIELVEAFAESX₆KQVTLVX₈X₉X₁₀DRILX₁₂ (SEQ ID No. 14) or GGGYIAIELVEAFAESX₆KQVTLVX₈X₉X₁₀DRILX₁₂ (SEQ ID No. 15), as a motif.

According to a particularly preferred embodiment of the present invention X₆ is glycine or asparagine and/or X₈ is aspartic acid or alanine and/or X₉ is glycine or arginine and/or X₁₀ is leucine or serine and/or X₁₂ is asparagine or arginine.

Surprisingly, it turned out that the substitution of some amino acid residues within the aforementioned motif allows to influence the substrate specificity of the protein of the present invention. The resulting protein variants may have a specificity for NADH, NADPH or even both.

In a particularly preferred embodiment of the present invention X₁₂ is asparagine. If the protein of the present invention comprises an asparagine residue at that position, the protein is able to oxidize NADH in a significantly higher degree as NADPH. Such a protein shows just a very reduced oxidation rate for NADPH. In another preferred embodiment of the present invention X₁₂ is arginine. If the protein of the present invention comprises an arginine residue at that position, the protein is able to oxidize NADH as well as NADPH at significant levels. This enables the proteins of the present invention to be used in complex reaction systems, where two or more lead enzymes perform reaction cascades having different cofactor (NADH, NADPH) preferences.

According to a further preferred embodiment of the present invention X₄ and X₆ are polar amino acid residues, preferably glycine or asparagine, X₈ is a non-polar amino acid residue, preferably alanine, X₁₀ is a polar amino acid residue, preferably serine, and X₉ and X₁₂ are a basic amino acid residue, preferably arginine. If the protein of the present invention comprises a motif with these amino acid residues, the protein is able to oxidize NADH to NAD⁺ and NADPH to NADP⁺. Such a protein can be advantageously used in reaction mixtures where either NADH or NADPH or even both need to be oxidized to NAD⁺ and/or NADP⁺, respectively. For instance, enzymatic reactions involving the reduction of NAD⁺ to NADH and/or NADP⁺ to NADPH may comprise such a protein for cofactor regeneration.

According to another preferred embodiment of the present invention X₄ is a non-polar amino acid residue, preferably alanine, X₆ is a polar amino acid residue, preferably glycine or asparagine, X₈ is a non-polar amino acid residue, preferably alanine, X₁₀ is a polar amino acid residue, preferably serine, and X₉ and X₁₂ are a basic amino acid residue, preferably arginine. Proteins of the present invention comprising these amino acid residues within the aforementioned motif are able to oxidize NADPH, thus forming NADP⁺.

According to a preferred embodiment of the present invention X₄ and X₆ are a polar amino acid residue, preferably glycine, X₈ is an acidic amino acid residue, preferably aspartic acid, X₉ is a polar amino acid residue, preferably glycine, X₁₀ is a non-polar amino acid residue, preferably leucine, and X₁₂ is a polar amino acid residue, preferably asparagine. If the aforementioned motif comprises these amino acid residues the protein of the present invention is able to oxidize NADH to NAD⁺.

According to another preferred embodiment of the present invention the protein of the present invention comprises an amino acid sequence having at least 85%, preferably at least 90%, more preferably at least 95%, more preferably at least 98%, in particular 100%, sequence identity with SEQ ID No. 1.

According to a preferred embodiment of the present invention the protein of the present invention comprises an amino acid sequence encoded by a nucleic acid sequence having an identity to SEQ ID No. 2 of at least 85%, preferably of at least 90%, more preferably of at least 95%, more preferably of at least 98%, in particular of 100%.

According to another preferred embodiment of the present invention the protein of the present invention comprises or consists of amino acid sequence SEQ ID No. 1, SEQ ID No. 16, SEQ ID No. 17, SEQ ID No. 18 or SEQ ID No. 19 or is encoded by a nucleic acid molecule comprising or consisting of SEQ ID No. 2, SEQ ID No. 20, SEQ ID No. 21, SEQ ID No. 22 or SEQ ID No. 23.
SEQ ID No. 16: 
MKVVVVGCTHAGTAAVKTILNEHPDASVSVYERNDNVSFLSCGIALYVGGVVKDPAGLFYSS
PEELASMGAKINMEHNVKNIDNENKVVVIENLKTGETFEESYDKLVMTTGSWPIIPPIDGIN
SENILLCKNYNQANEIIKESKNAKKIVIVGGGYIGIELVEAFAESGKQVTLVARSDRILRKY
LDAEFTSVLEHDLQERGVTLALNQTVEKFVANESGAVTAVKTPVGEYEADLVILCVGFKPNT
DLLKDKVEMLPNGAIVVDEYMRTSDEAIFAAGDSCAVHYNPTGGSAYIPLATNAVRMGALVG
KNIVSPTVKYRGTQATSGLYLFGFNIGSTGLTENSAPHFGVEVRSVVVEDNYRPEFMPTTEK
VTMKLVYEVGTNRIVGGQIMSKYDVTQSANTLSLCVQNKMTIEDLAYVDFFFQPHFDRPWNY
LNILAQAAVEQERKLAK
SEQ ID No. 20: 
ATGAAAGTAGTAGTAGTAGGCTGTACACATGCAGGAACAGCGGCAGTTAAGACGATTTTA
AATGAACATCCAGATGCATCAGTATCAGTATATGAGCGTAATGACAATGTCTCATTTCTA
TCTTGTGGGATTGCGTTGTATGTTGGTGGAGTTGTGAAAGATCCTGCAGGTTTGTTTTAT
TCAAGTCCAGAAGAACTTGCATCAATGGGCGCGAAAATTAACATGGAACACAATGTGAAA
AATATAGATAATGAGAATAAGGTCGTAGTAATTGAGAATTTAAAAACAGGCGAAACATTT
GAAGAAAGCTATGATAAGTTGGTAATGACAACTGGATCATGGCCAATTATTCCTCCAATT
GATGGAATCAATAGTGAAAATATTCTTTTGTGTAAAAACTATAACCAAGCAAATGAAATT
ATTAAAGAATCAAAAAATGCTAAAAAGATTGTCATTGTTGGTGGTGGCTATATTGGAATT
GAATTAGTTGAGGCATTTGCAGAATCTGGCAAGCAAGTGACGCTAGTTGCGCGTAGCGAT
CGTATTTTACGTAAATATTTAGATGCTGAATTCACTTCTGTTTTAGAGCATGATTTACAA
GAAAGAGGCGTTACGCTAGCTTTAAACCAAACCGTCGAGAAATTTGTTGCCAATGAATCA
GGTGCTGTGACAGCTGTGAAAACACCAGTTGGAGAATATGAGGCTGATTTAGTTATTTTA
TGTGTTGGATTTAAACCAAATACTGATTTGTTGAAGGATAAAGTAGAGATGTTGCCAAAT
GGTGCCATCGTAGTGGATGAATATATGAGAACAAGCGATGAAGCGATTTTTGCTGCTGGC
GATAGTTGCGCGGTTCATTATAATCCAACTGGAGGCTCTGCGTATATTCCGTTAGCTACA
AATGCAGTTAGAATGGGAGCTTTAGTTGGAAAAAATATTGTTTCTCCAACAGTTAAATAT
CGTGGCACGCAAGCAACTTCTGGTTTATATTTATTTGGTTTTAATATAGGTTCAACCGGA
 
TTGACTGAAAATAGCGCTCCTCATTTTGGCGTAGAGGTTCGTTCAGTAGTTGTAGAAGAT
AATTATCGTCCAGAGTTTATGCCGACAACAGAGAAAGTAACGATGAAATTAGTTTATGAA
GTAGGAACGAATCGGATTGTTGGAGGTCAAATCATGTCAAAATATGATGTGACACAATCT
GCCAATACGTTATCTTTATGTGTTCAAAATAAAATGACGATTGAGGATTTGGCTTATGTA
GATTTCTTCTTCCAACCTCACTTTGATCGTCCTTGGAACTATTTAAATATTTTAGCGCAA
GCAGCTGTTGAGCAAGAGCGTAAACTAGCAAAATAA
SEQ ID No. 17: 
MKVVVVGCTHAGTAAVKTILNEHPDASVSVYERNDNVSFLSCGIALYVGGVVKDPAGLFYSS
PEELASMGAKINMEHNVKNIDNENKVVVIENLKTGETFEESYDKLVMTTGSWPIIPPIDGIN
SENILLCKNYNQANEIIKESKNAKKIVIVGGGYIAIELVEAFAESGKQVTLVARSDRILRKY
LDAEFTSVLEHDLQERGVTLALNQTVEKFVANESGAVTAVKTPVGEYEADLVILCVGFKPNT
DLLKDKVEMLPNGAIVVDEYMRTSDEAIFAAGDSCAVHYNPTGGSAYIPLATNAVRMGALVG
KNIVSPTVKYRGTQATSGLYLFGFNIGSTGLTENSAPHFGVEVRSVVVEDNYRPEFMPTTEK
VTMKLVYEVGTNRIVGGQIMSKYDVTQSANTLSLCVQNKMTIEDLAYVDFFFQPHFDRPWNY
LNILAQAAVEQERKLAK
SEQ ID No. 21: 
ATGAAAGTAGTAGTAGTAGGCTGTACACATGCAGGAACAGCGGCAGTTAAGACGATTTTA
AATGAACATCCAGATGCATCAGTATCAGTATATGAGCGTAATGACAATGTCTCATTTCTA
TCTTGTGGGATTGCGTTGTATGTTGGTGGAGTTGTGAAAGATCCTGCAGGTTTGTTTTAT
TCAAGTCCAGAAGAACTTGCATCAATGGGCGCGAAAATTAACATGGAACACAATGTGAAA
AATATAGATAATGAGAATAAGGTCGTAGTAATTGAGAATTTAAAAACAGGCGAAACATTT
GAAGAAAGCTATGATAAGTTGGTAATGACAACTGGATCATGGCCAATTATTCCTCCAATT
GATGGAATCAATAGTGAAAATATTCTTTTGTGTAAAAACTATAACCAAGCAAATGAAATT
ATTAAAGAATCAAAAAATGCTAAAAAGATTGTCATTGTTGGTGGTGGCTATATTGCGATT
GAATTAGTTGAGGCATTTGCAGAATCTGGCAAGCAAGTGACGCTAGTTGCGCGTAGCGAT
CGTATTTTACGTAAATATTTAGATGCTGAATTCACTTCTGTTTTAGAGCATGATTTACAA
GAAAGAGGCGTTACGCTAGCTTTAAACCAAACCGTCGAGAAATTTGTTGCCAATGAATCA
GGTGCTGTGACAGCTGTGAAAACACCAGTTGGAGAATATGAGGCTGATTTAGTTATTTTA
TGTGTTGGATTTAAACCAAATACTGATTTGTTGAAGGATAAAGTAGAGATGTTGCCAAAT
GGTGCCATCGTAGTGGATGAATATATGAGAACAAGCGATGAAGCGATTTTTGCTGCTGGC
GATAGTTGCGCGGTTCATTATAATCCAACTGGAGGCTCTGCGTATATTCCGTTAGCTACA
AATGCAGTTAGAATGGGAGCTTTAGTTGGGAAAAATATTGTTTCTCCAACAGTTAAATAT
CGTGGCACGCAAGCAACTTCTGGTTTATATTTATTTGGTTTTAATATAGGTTCAACCGGA
TTGACTGAAAATAGCGCTCCTCATTTTGGCGTAGAGGTTCGTTCAGTAGTTGTAGAAGAT
AATTATCGTCCAGAGTTTATGCCGACAACAGAGAAAGTAACGATGAAATTAGTTTATGAA
GTAGGAACGAATCGGATTGTTGGAGGTCAAATCATGTCAAAATATGATGTGACACAATCT
 
GCCAATACGTTATCTTTATGTGTTCAAAATAAAATGACGATTGAGGATTTGGCTTATGTA
GATTTCTTCTTCCAACCTCACTTTGATCGTCCTTGGAACTATTTAAATATTTTAGCGCAA
GCAGCTGTTGAGCAAGAGCGTAAACTAGCAAAATAA
SEQ ID No. 18: 
MKVVVVGCTHAGTAAVKTILNEHPDASVSVYERNDNVSFLSCGIALYVGGVVKDPAGLFYSS
PEELASMGAKINMEHNVKNIDNENKVVVIENLKTGETFEESYDKLVMTTGSWPIIPPIDGIN
SENILLCKNYNQANEIIKESKNAKKIVIVGGGYIGIELVEAFAESNKQVTLVARSDRILRKY
LDAEFTSVLEHDLQERGVTLALNQTVEKFVANESGAVTAVKTPVGEYEADLVILCVGFKPNT
DLLKDKVEMLPNGAIVVDEYMRTSDEAIFAAGDSCAVHYNPTGGSAYIPLATNAVRMGALVG
KNIVSPTVKYRGTQATSGLYLFGFNIGSTGLTENSAPHFGVEVRSVVVEDNYRPEFMPTTEK
VTMKLVYEVGTNRIVGGQIMSKYDVTQSANTLSLCVQNKMTIEDLAYVDFFFQPHFDRPWNY
LNILAQAAVEQERKLAK
SEQ ID No. 22: 
ATGAAAGTAGTAGTAGTAGGCTGTACACATGCAGGAACAGCGGCAGTTAAGACGATTTTA
AATGAACATCCAGATGCATCAGTATCAGTATATGAGCGTAATGACAATGTCTCATTTCTA
TCTTGTGGGATTGCGTTGTATGTTGGTGGAGTTGTGAAAGATCCTGCAGGTTTGTTTTAT
TCAAGTCCAGAAGAACTTGCATCAATGGGCGCGAAAATTAACATGGAACACAATGTGAAA
AATATAGATAATGAGAATAAGGTCGTAGTAATTGAGAATTTAAAAACAGGCGAAACATTT
GAAGAAAGCTATGATAAGTTGGTAATGACAACTGGATCATGGCCAATTATTCCTCCAATT
GATGGAATCAATAGTGAAAATATTCTTTTGTGTAAAAACTATAACCAAGCAAATGAAATT
ATTAAAGAATCAAAAAATGCTAAAAAGATTGTCATTGTTGGTGGTGGCTATATTGGAATT
GAATTAGTTGAGGCATTTGCAGAATCTAACAAGCAAGTGACGCTAGTTGCGCGTAGCGAT
CGTATTTTACGTAAATATTTAGATGCTGAATTCACTTCTGTTTTAGAGCATGATTTACAA
GAAAGAGGCGTTACGCTAGCTTTAAACCAAACCGTCGAGAAATTTGTTGCCAATGAATCA
GGTGCTGTGACAGCTGTGAAAACACCAGTTGGAGAATATGAGGCTGATTTAGTTATTTTA
TGTGTTGGATTTAAACCAAATACTGATTTGTTGAAGGATAAAGTAGAGATGTTGCCAAAT
GGTGCCATCGTAGTGGATGAATATATGAGAACAAGCGATGAAGCGATTTTTGCTGCTGGC
GATAGTTGCGCGGTTCATTATAATCCAACTGGAGGCTCTGCGTATATTCCGTTAGCTACA
AATGCAGTTAGAATGGGAGCTTTAGTTGGGAAAAATATTGTTTCTCCAACAGTTAAATAT
CGTGGCACGCAAGCAACTTCTGGTTTATATTTATTTGGTTTTAATATAGGTTCAACCGGA
TTGACTGAAAATAGCGCTCCTCATTTTGGCGTAGAGGTTCGTTCAGTAGTTGTAGAAGAT
AATTATCGTCCAGAGTTTATGCCGACAACAGAGAAAGTAACGATGAAATTAGTTTATGAA
GTAGGAACGAATCGGATTGTTGGAGGTCAAATCATGTCAAAATATGATGTGACACAATCT
GCCAATACGTTATCTTTATGTGTTCAAAATAAAATGACGATTGAGGATTTGGCTTATGTA
GATTTCTTCTTCCAACCTCACTTTGATCGTCCTTGGAACTATTTAAATATTTTAGCGCAA
GCAGCTGTTGAGCAAGAGCGTAAACTAGCAAAATAA
SEQ ID No. 19: 
MKVVVVGCTHAGTAAVKTILNEHPDASVSVYERNDNVSFLSCGIALYVGGVVKDPAGLFYSS
PEELASMGAKINMEHNVKNIDNENKVVVIENLKTGETFEESYDKLVMTTGSWPIIPPIDGIN
SENILLCKNYNQANEIIKESKNAKKIVIVGGGYIAIELVEAFAESNKQVTLVARSDRILRKY
LDAEFTSVLEHDLQERGVTLALNQTVEKFVANESGAVTAVKTPVGEYEADLVILCVGFKPNT
DLLKDKVEMLPNGAIVVDEYMRTSDEAIFAAGDSCAVHYNPTGGSAYIPLATNAVRMGALVG
KNIVSPTVKYRGTQATSGLYLFGFNIGSTGLTENSAPHFGVEVRSVVVEDNYRPEFMPTTEK
VTMKLVYEVGTNRIVGGQIMSKYDVTQSANTLSLCVQNKMTIEDLAYVDFFFQPHFDRPWNY
LNILAQAAVEQERKLAK
SEQ ID No. 23:

ATGAAAGTAGTAGTAGTAGGCTGTACACATGCAGGAACAGCGGCAGTTAAGACGATTTTA
AATGAACATCCAGATGCATCAGTATCAGTATATGAGCGTAATGACAATGTCTCATTTCTA
TCTTGTGGGATTGCGTTGTATGTTGGTGGAGTTGTGAAAGATCCTGCAGGTTTGTTTTAT
TCAAGTCCAGAAGAACTTGCATCAATGGGCGCGAAAATTAACATGGAACACAATGTGAAA
AATATAGATAATGAGAATAAGGTCGTAGTAATTGAGAATTTAAAAACAGGCGAAACATTT
GAAGAAAGCTATGATAAGTTGGTAATGACAACTGGATCATGGCCAATTATTCCTCCAATT
GATGGAATCAATAGTGAAAATATTCTTTTGTGTAAAAACTATAACCAAGCAAATGAAATT
ATTAAAGAATCAAAAAATGCTAAAAAGATTGTCATTGTTGGTGGTGGCTATATTGCGATT
GAATTAGTTGAGGCATTTGCAGAATCTAACAAGCAAGTGACGCTAGTTGCGCGTAGCGAT
CGTATTTTACGTAAATATTTAGATGCTGAATTCACTTCTGTTTTAGAGCATGATTTACAA
GAAAGAGGCGTTACGCTAGCTTTAAACCAAACCGTCGAGAAATTTGTTGCCAATGAATCA
GGTGCTGTGACAGCTGTGAAAACACCAGTTGGAGAATATGAGGCTGATTTAGTTATTTTA
TGTGTTGGATTTAAACCAAATACTGATTTGTTGAAGGATAAAGTAGAGATGTTGCCAAAT
GGTGCCATCGTAGTGGATGAATATATGAGAACAAGCGATGAAGCGATTTTTGCTGCTGGC
GATAGTTGCGCGGTTCATTATAATCCAACTGGAGGCTCTGCGTATATTCCGTTAGCTACA
AATGCAGTTAGAATGGGAGCTTTAGTTGGGAAAAATATTGTTTCTCCAACAGTTAAATAT
CGTGGCACGCAAGCAACTTCTGGTTTATATTTATTTGGTTTTAATATAGGTTCAACCGGA
TTGACTGAAAATAGCGCTCCTCATTTTGGCGTAGAGGTTCGTTCAGTAGTTGTAGAAGAT
AATTATCGTCCAGAGTTTATGCCGACAACAGAGAAAGTAACGATGAAATTAGTTTATGAA
GTAGGAACGAATCGGATTGTTGGAGGTCAAATCATGTCAAAATATGATGTGACACAATCT
GCCAATACGTTATCTTTATGTGTTCAAAATAAAATGACGATTGAGGATTTGGCTTATGTA
GATTTCTTCTTCCAACCTCACTTTGATCGTCCTTGGAACTATTTAAATATTTTAGCGCAA
GCAGCTGTTGAGCAAGAGCGTAAACTAGCAAAATAA

The protein comprising or consisting of amino acid sequence SEQ ID No. 1 or encoded by nucleic acid sequence SEQ ID No. 2 is able to oxidize NADH. The protein comprising or consisting of amino acid sequence SEQ ID No. 17 or encoded by nucleic acid sequence SEQ ID No. 21 is able to oxidize NADPH. The proteins comprising or consisting of amino acid sequence SEQ ID No. 16, SEQ ID No. 18 and SEQ ID No. 19 or encoded by nucleic acid sequence SEQ ID No. 20, SEQ ID No. 22 and SEQ ID No. 23 are able to oxidize NADH as well as NADPH.

The protein of the present invention can be modified once, twice, three, four or five times by a water-soluble polymer. A water-soluble polymer is polyethylene glycol, for example. The binding of the polyethylene glycol preferably takes place at the N-terminal end of the protein according to the present invention. The protein of the present invention can also be bound to a solid body such as polyethylene, polystyrene, polysaccharide, cellulose or cellulose derivatives.

According to a preferred embodiment of the present invention the protein of the present invention maybe truncated at the N-and/or C-terminus. A truncated protein of the present invention may comprise or consist of 440 to 450 amino acid residues.

Furthermore, the protein of the present invention may be part of a fusion protein. Such a fusion protein may comprise next to the protein of the present invention at least a further polypeptide at the N-terminal and/or C-terminal end. Fusion proteins can, for example, be separated more easily from other proteins or are expressed in the cells in larger amounts.

A further aspect of the present invention relates to a nucleic acid molecule encoding a protein according to the present invention. The nucleic acid molecule may be a DNA or an RNA molecule.

According to a preferred embodiment of the present invention the nucleic acid molecule of the present invention
i) comprises or consists of a nucleic acid sequence having an identity to SEQ ID No. 2 of at least 80%, or ii) binds under stringent conditions to a nucleic acid molecule complementary to the nucleic acid sequence SEQ ID No. 2, wherein the stringent conditions preferably comprise washing at 65°C, and at a salt concentration of 0.1 to 2x SSC.

According to another preferred embodiment of the present invention the nucleic acid molecule comprises or consists of nucleic acid sequence SEQ ID No. 2, SEQ ID No. 20, SEQ ID No. 21, SEQ ID No. 22 or SEQ ID No. 23.

A further aspect of the present invention relates to a vector comprising a nucleic acid molecule according to the present invention.

The term "vector", as used herein, refers to a nucleic acid molecule capable of transporting another nucleic acid to which it is linked. Useful vectors are those capable of autonomous replication and/or expression of the nucleic acid linked to it. Vectors capable of directing the expression of genes to which they are operatively linked are referred to herein as "expression vectors". Expression vectors used in recombinant DNA technology often have the form of a "plasmid", which usually refers to a circular double-stranded DNA loop that does not bind to a chromosome when in the form of a vector. As used herein, "plasmid" and "vector" are used interchangeably because plasmids are the most commonly used form of vector. However, other forms of expression vectors that perform equivalent functions and are subsequently known in the art are also included.

Suitable cloning vectors for overexpression and production of the proteins of the present invention include, for example, pKK223-3, pTrc99a, pUC, pTZ, pSK, pBluescript, pGEM, pQE, pET, PHUB, pPLc, pKC30, pRM1/pRM9, pTrxFus, pAS1, pGEx, pMAL or pTrx.

The nucleic acid molecule of the present invention encoding the protein of the present invention may be operably linked to a promoter. The term "operably linked", as used herein, means that a selected nucleotide sequence (such as encoding a protein described herein) is in close proximity to the promoter to allow the promoter to regulate the expression of the selected nucleic acid molecule. In addition, the promoter is located upstream of the selected nucleotide sequence in the direction of transcription and translation. By "operably linked" is meant that when an appropriate molecule (such as a transcriptional activator protein) is bound to a regulatory sequence, the nucleotide sequence and the regulatory sequence are linked in such a way that the nucleic acid molecule/gene is expressed.

Suitable expression promoters include, for example, trp-lac (tac)-promotor, trp-lac (trc) promotor, lac-promotor, T7-promotor and λpL-promotor.

Another aspect of the present invention relates to a host cell comprising a nucleic acid molecule or a vector according to the present invention.

Host cells comprising a nucleic acid molecule or a vector according to the present invention can be used for producing the protein of the present invention. Suitable host cells include bacteria like *E. coli* and yeast cells like *Komagataella phaffii.*

A further aspect of the present invention relates to the use of a protein or a host cell according to the present invention or a lysate or homogenate thereof for oxidizing NADH to NAD⁺ and/or NADPH to NADP⁺.

The protein of the present invention can be used in any method for oxidizing NADH to NAD⁺ and/or NADPH to NADP⁺. The protein may be an isolated protein (e.g. from a host cells as mentioned above) or an isolated immobilized protein or may be part of whole cell or an immobilized whole cell and a cell homogenate or a cell lysate. The use of cell lysates is particularly preferred because lysates comprise cofactors NADH/NAD⁺ and NADPH/NADP⁺ so that in complex reaction mixtures involving oxidoreductases, for instance, it is not necessary to add further cofactors.

Another aspect of the present invention relates to a method for oxidizing NADH to NAD⁺ and/or NADPH to NADP⁺ comprising the step of incubating a protein or a host cell according to the present invention or a lysate or homogenate thereof with NADH and/or NADPH.

A further aspect of the present invention relates to the use of a protein or a host cell according to the present invention or a lysate or homogenate thereof in a cofactor recycling system or as a cofactor recycling system.

The protein of the present invention can be used in cofactor recycling systems where NADH is recycled to NAD⁺ and/or NADPH is recycled to NADP⁺. Such cofactor recycling systems are well-known to the person skilled in the art. The protein of the present invention can also be used as a cofactor recycling system in reactions where NADH has to be recycled to NAD⁺ and/or NADPH has to be recycled to NADP⁺.

Another aspect of the present invention relates to a method for the enzymatic oxidation of a compound, where NADH is formed from NAD⁺ and/or NADPH is formed from NADP⁺, comprising the step of oxidizing NADH to NAD⁺ and/or NADPH to NADP⁺ by adding a protein according to the present invention or a host cell according to the present invention or a lysate or homogenate of said host cell.

The temperature optimum of the protein of the present invention lies between 20°C and 45°C and the optimum pH between 6.0 and 8.5. The protein shows good temperature and pH stabilities and is stable for more than 80 hours when incubated at pH 7.0 and 30 °C. Hence, the protein of the present invention is much more stable compared to other NAD(P)H oxidases known in the art. Furthermore, it surprisingly exhibits high stability in aqueous solutions comprising organic co-solvents, such as acetone and isopropanol. It could be shown that the protein of the present invention is stable and enzymatically active in aqueous solutions comprising up to 25% (v/v), preferably up to 20% (v/v), more preferably up to 15% (v/v), of an organic solvent like an alcohol, such as isopropanol, or acetone. Hence, it is particularly preferred to use the protein of the present invention in aqueous reaction mixtures comprising the aforementioned amounts of organic solvents or in aqueous reaction mixtures comprising 0.5 to 25% (v/v), preferably 1 to 20% (v/v), more preferably 1 to 15% (v/v), of at least one organic cosolvent. Under such conditions the proteins of the present invention are enzymatically active for a minimum of 20 h at 30°C.

The protein of the present invention can be used in the method of the present invention either as whole cells, cell homogenate, cell lysate, or in a completely (at least 95%, preferably at least 98%) or partially purified state. The method of the present invention is carried out with the protein according to the invention or with cells containing the protein according to the invention. In doing so, the cells used can be provided in a native, permeabilized or lysed state. Preferably, the proteins comprising or consisting of the amino acid sequences SEQ ID No. 1, SEQ ID No. 16, SEQ ID No. 17, SEQ ID No. 18 or SEQ ID No. 19 are used.

### Method for reducing/oxidizing bile acids

Due to the outstanding stability and enzymatic activity of the NAD(P)H oxidase of the present invention in the presence of organic solvents, theses enzymes can be used in oxidation and/or reduction reactions involving bile acids like cholic acid and derivatives thereof.

Thus, another aspect of the present invention relates to a method of incubating bile acids with NAD(P)H dependent Oxidoreductases. In a particular aspect of the present invention cholic acid is incubated with 12α-hydroxysteroid dehydrogenase and/or 7α-hydroxysteroid dehydrogenase and the NAD(P)H oxidase of the present invention in the presence of NADH/NAD⁺ to form 7-keto-deoxycholic acid, 12-keto-chenodeoxycholic acid and/or 7,12-diketo-lithocholic acid.

### Method for the preparation of an aqueous solution containing D-psicose or L-psicose

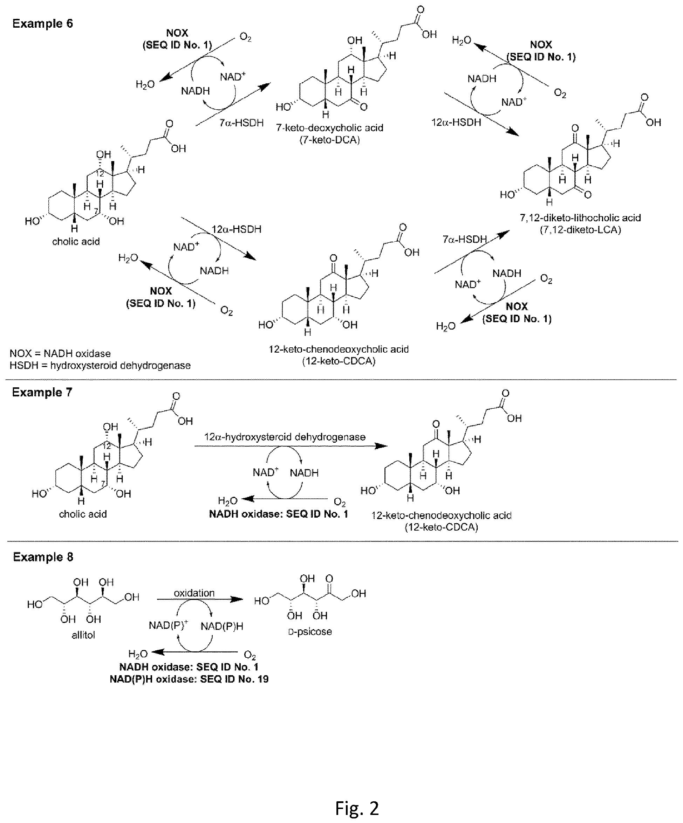

Another aspect of the present invention aims to provide an efficient and environmentally friendly process for the preparation of aqueous solutions containing D-psicose or L-psicose with high conversion, which can be carried out in particular in a one-pot process. This process comprises the step of forming a first D-psicose from D-fructose, which is present dissolved in an aqueous solution, by treatment with an epimerase *in vitro,* whereafter the first D-psicose is reduced to allitol by treatment with an NAD(P)H-dependent oxidoreductase *in vitro* and, after deactivation of the epimerase to form D-psicose or L-psicose, is treated with a corresponding NAD(P)-dependent oxidoreductase, whereafter the deactivated epimerase and the oxidoreductases are removed. The formation of D-psicose from allitol is shown in Fig. 2.

Surprisingly, it has been shown that the reactions can be carried out with enzymes that are contained in an aqueous solution, i.e. *in vitro.*

A preferred variant of the process according to the invention is that the oxidized cofactor NAD(P)+ formed by the reduction of D-psicose to allitol is reduced by means of an alcohol dehydrogenase (ADH) and a secondary alcohol to form a ketone, the secondary alcohol preferably being 2-propanol (isopropanol). 2-Propanol is a very favorable hydrogen donor for the regeneration of NAD(P)H and the oxidation product acetone is easily separable due to its volatility. Acetone recovered from the exhaust stream can be heterogeneously catalyzed in the gas phase to be hydrogenated back to 2-propanol, and hydrogen from sustainable sources ("green hydrogen") could be increasingly used in the future. The use of such a system allows the recycling of 2-propanol without the emission of climate-damaging CO₂ and also avoids large amounts of waste (such as unreacted sodium formate in the FDH regeneration system).

Regeneration of the cofactor using ADH is, for example, previously known from EP 2 812 439.

Another preferred variant of this process is characterized in that it is carried out as a one-pot reaction without isolation of intermediates.

The enzymes used for producing D-psicose and/or L-psicose are preferably used as lysate of the corresponding cells forming them. The enzymes are preferably expressed individually in suitable *E. coli* production strains, for instance. This allows optimization of the individual expression levels of the enzymes.

Before performing the last step (oxidation), the enzymes (epimerase and reductase and/or dehydrogenase) of the first step (D-fructose to allitol) are deactivated by heat and/or removed by ultrafiltration to prevent the formation of by-products by the enzymes. Especially in the case of D-psicose, without the appropriate treatment, much of the D-psicose formed by oxidation would be converted back to D-fructose by epimerase.

The regeneration of nicotinamide-based cofactors (NAD or NADP) occurs with an NAD(P)-dependent alcohol dehydrogenase in the case of the reduction of D-psicose to allitol and with an H₂O-forming NAD(P)H oxidase in the case of the oxidation reactions (second step).

The particularly preferred concentration of D-fructose is 50 - 250 g/l.

The particularly preferred temperature range for the first step (epimerization and reduction) is between 25 and 45 °C, for the second step (oxidation) between 20 and 30 °C.

The particularly preferred pH range for both steps is between 7 and 8.5.

The enzymes may be present in a suspension, in the homogenate and/or in the lysate of the corresponding cells forming them, lysates being particularly preferred.

Suspension in this context means a suspension of resting cells. These are harvested (separated from the culture medium) after cultivation and used as a paste or suspended in a suitable buffer system. In contrast to fermentative processes, which also work with whole cells, the resting cells can no longer grow due to the absence of carbon sources and nutrients, but only serve to convert substrates. Homogenate in this context stands for a physically and/or chemically treated suspension (e.g., treated by pressure, lysozyme, or ultrasound), releasing cellular components from the cells. A lysate is obtained when the insoluble cellular components of the homogenate are removed by, for example, filtration or centrifugation.

In a further embodiment, the enzymes may also be immobilized on a solid support material.

In a particularly preferred embodiment of the process, only enzymes from the enzyme groups epimerases, dehydrogenases, reductases and oxidases are used for the conversion of the starting material, with one or more of these enzymes being selected from each of these groups.

The epimerase used in the process may be from one of the groups EC 5.1.3.30 (D-psicose 3-epimerase) or EC 5.1.3.31 (D-tagatose 3-epimerase/L-ribulose 3-epimerase), the former being particularly preferred.

The enzyme used for the reduction of D-psicose and the oxidation of allitol are from the group of oxidoreductases (see Table 1 for details).

The alcohol dehydrogenase (ADH) used for cofactor regeneration may be from one of the groups EC 1.1.1.1 (NAD-dependent ADH) and EC 1.1.1.2 (NADP-dependent ADH).

The NAD(P)H oxidase used for cofactor regeneration may be from one of the groups EC 1.6.3.1 (NAD(P)H oxidase (H₂O₂-forming)), EC 1.6.3.2 (NAD(P)H oxidase (H2O-forming)), EC 1. 6.3.3 (NADH oxidase (H₂O₂-forming)) and EC 1.6.3.4 (NADH oxidase (H₂O-forming)), the H₂O-forming classes being preferred. The NAD(P)H oxidase used for cofactor regeneration in this method for producing D-psicose and/or L-psicose is the the NAD(P)H oxidase of the present invention.

### EXAMPLES

### Example 1

### Cloning of NADH oxidase from Carnobacterium divergens

Based on Seq ID No. 2, available in the NCBI genomic database [JQBS01000032.1], specific primers were constructed for a subsequent cloning of the full-length gene into appropriate expression systems. In doing so, 5'-primers having a recognition sequence for SphI and 3'-primers having a recognition sequence for HindIII were modified.

Total genomic DNA isolated from fresh cells of *Carnobacterium divergens* (DSMZ 20625) served as a template for the polymerase chain reaction. The resulting fragment of about 1500 nucleotides was digested with endonucleases SphI and HindIII and was ligated into the backbone of the pQE vector series (Qiagen), which backbone had been treated with the same endonucleases. After transforming 2 µl of the ligation batch into *E. coli* Top 10 F' cells (Invitrogen), plasmid DNAs of kanamycin-resistant colonies were tested for the presence of an insert having a size of 1,500 bp by using a restriction analysis with endonucleases SphI and HindIII. The resulted expression constructs A or B and C were sequenced and used to transform the expression host strains of *Escherichia coli.*

### Example 2

### Expression of the NADH oxidase according to the invention from Carnobacterium divergens in RB791 E. coli cells

Competent *Escherichia coli* RB791 cells (Coli Genetic Stock Center, Yale) were transformed with the expression constructs A or B and C coding for NADH oxidase according to the invention.

The transformed strain was cultivated in an LB medium (1% tryptone, 0.5% yeast extract, 1% NaCl) with kanamycin (50 pg/ml) until an optical density of 0.5 (measured at 550 nm) was reached. The production of the recombinant oxidoreductase protein was initiated by adding isopropylthiogalactoside (IPTG) at a final concentration of 1 mM. The induction batch was incubated for another 19 h at 25 °C and 160 rpm.

The enzyme activity achieved with this procedure amounted to approx. 3,000 U/g of wet cell mass.

### Example 3

### Stability of the recombinant NADH oxidase

The stability of the recombinant NADH oxidase according to the invention was determined in different buffer systems at 25 °C and 850 rpm for at least 90 h. For this purpose, a 1:10 dilution of the 20 % w/v homogenate of recombinant NADH oxidase according to the invention was incubated in different buffer systems and was measured photometrically at 340 nm in the following reaction mixture at 30 °C: 890 µl of 100 mM potassium phosphate buffer (pH 7.0) and 10 µl of enzyme dilution. The reaction was started by addition of 100 µl of a 2 mM NADH or NADPH solution in H₂O. The measured value obtained immediately after diluting the NADH oxidase in a potassium phosphate buffer (50 mM, pH 7.0) was used as the initial value (100%).

This example shows that the enzyme is stable at a temperature 25 °C and even after 90 h of incubation 75 % initial activity could be detected, without addition of stabilizers and/or other antioxidative agents.

### Example 4

### Comparison of stability of the recombinant NADH oxidase according to the invention with the most stable known NADH oxidase from Streptococcus mutans (EMC51149) (UniProt, Q54453)

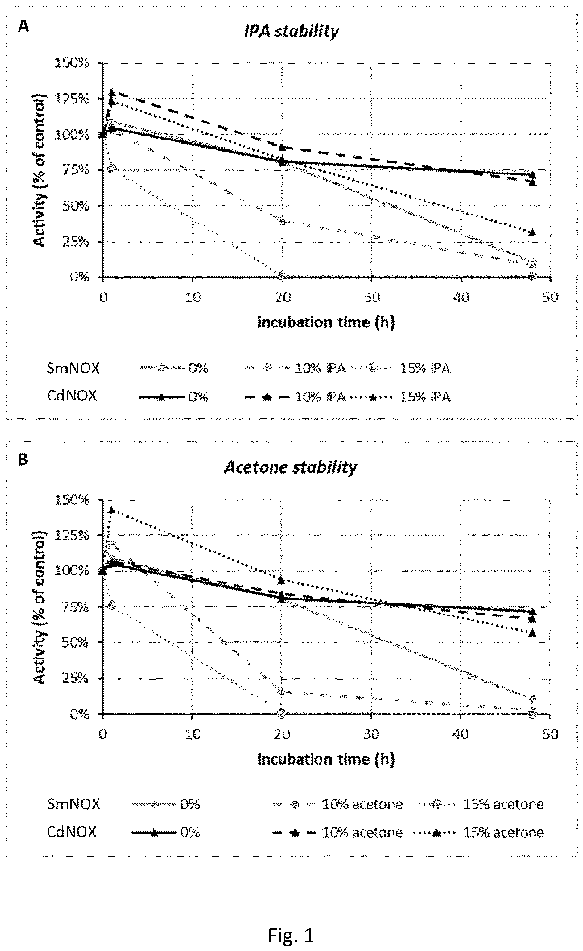

The stability of the recombinant NADH oxidase according to the invention as well as the stability of recombinant NADH oxidase from *Streptococcus mutans* were determined in triethanolamine (TEA) buffer at 25 °C and 850 rpm for 48 h. For this purpose, a 1:10 dilution of the 20 % w/v homogenate of recombinant NADH oxidase according to the invention was incubated as indicated (see Table 1) in the presence of increasing values of acetone or isopropanol, respectively, and were measured photometrically at 340 nm in the following reaction mixture at 25°C: 890 µl of 100 mM potassium phosphate buffer 100 mM (pH 7.0) and 10 µl of enzyme dilution. The reaction was started by addition of 100 µl of a 2 mM NADH solution in H₂O. The measured value obtained immediately after diluting the NADH oxidase in a potassium phosphate buffer (100 mM, pH 7.0) was used as the initial value (100%). The results of the measurements are displayed in Table 1 and Figure 1.

**Table 1. Results of the stability experiments for the NADH oxidases from Streptococcus mutans and Carnobacterium divergens. The activities of the NADH oxidases from both microorganisms were measured after 1, 20 and 48 h and are given in percentages referred to the activity measured at the beginning of the experiment.**

| | NADH oxidasefrom *Streptococcus mutans* | | | NADH oxidasefrom *Carnobacterium divergens* | | |
|---|---|---|---|---|---|---|
| **organic solvent** | **1 h** | **20 h** | **48 h** | **1 h** | **20 h** | **48 h** |
| 0% | 109% | 81% | **10%** | 105% | 81% | **72%** |
| 10% IPA | 103% | 39% | **9%** | 130% | 91% | **67%** |
| 15% IPA | 76% | 1% | 1% | 123% | 83% | 31% |
| 10% acetone | 119% | 16% | **3%** | 106% | 84% | **67%** |
| 15% acetone | 76% | 1% | 0% | 143% | 94% | 57% |

This example clearly demonstrates that the NADH oxidase according to the invention is more stable than the NADH oxidase from *Streptococcus mutans.* For example after 48 h of incubation at 25 °C and 850 rpm without addition of stabilizers, the NADH oxidase according to the invention retained even 72/67/67% (without org. solvent/10% IPA/10% acetone) of initial activity compared to 10/9/3% (without org. solvent/10% IPA/10% acetone) of initial activity in case of the NADH oxidase from *Streptococcus mutans.*

### Example 5

### Comparison of cholic acid resistance of the recombinant NADH oxidase according to the invention with the most stable known NADH oxidase from Streptococcus mutans (EMC51149) (UniProt, Q54453)

Cholic acid (CA) is a protein unfolding and protein aggregation-inducing agent in bacteria (Cremers et al., 2014). The stability of the recombinant NADH oxidase according to the invention as well as the stability of recombinant NADH oxidase from *Streptococcus mutans* was determined in CA-containing buffer at 25 °C and 850 rpm for 20 h. For this purpose, a 1:10 dilution of the 20 % w/v homogenate of recombinant NADH oxidase according to the invention was incubated in 100 mM KPP pH 8.0, 10% glycerine and 5% CA and were measured photometrically at 340 nm in the following reaction mixture at 25°C: 890 µl of 100 mM potassium phosphate buffer 100 mM (pH 7.0) and 10 µl of enzyme dilution. The reaction was started by addition of 100 µl of a 2 mM NADH solution in H₂O. The measured value obtained immediately after diluting the NADH oxidase in a potassium phosphate buffer (100 mM, pH 7.0) was used as the initial value (1000).

**Table 2. Results of the CA-stability experiments for the NADH oxidases from Streptococcus mutans and Carnobacterium divergens. The activities of the NADH oxidases from both microorganisms were measured after 1 h, 17 h and 20 h and are given in percentages referred to the activity measured at the beginning of the experiment.**

| NADH oxidase from *Streptococcus mutans* | | | NADH oxidase from *Carnobacterium divergens* | | |
|---|---|---|---|---|---|
| **1 h** | **17 h** | **20 h** | **1 h** | **17 h** | **20 h** |
| 93% | 21% | **19%** | 103% | 50% | **45%** |

This example clearly demonstrates that the NADH oxidase according to the invention is more stable than the NADH oxidase from *Streptococcus mutans* in presence of CA, a protein unfolding and protein aggregation inducing agent. Note that after 20 h incubation, the NADH oxidase according to the invention shows twice as much remaining activity compared to the NADH oxidase from *Streptococcus mutans.*

### Example 6

### Synthesis of 7,12-diketo-lithocholic acid (7,12-diketo-LCA) from cholic acid (CA) using NADH oxidase according to SEQ ID No. 1 as cofactor regeneration enzyme

To 25 mg cholic acid (50 g/l substrate load) incubated in 50 mM triethanolamine buffer (pH 8.0) containing 8.5 v% glycerol and 0.3 mM NAD⁺, 5 U of 12α-hydroxysteroid dehydrogenase from *Eggerthella lenta* (UniProt, C8WLK7), 2.6 U of 7α-hydroxysteroid dehydrogenase from *Escherichia coli* (UniProt, P0AET8) and 3 U of different recombinant NADH oxidases (applied as cell suspension formulation) were added (see Table 3 below). The reaction mixtures were incubated for 20 h at 22 °C and 850 rpm (Eppendorf Thermomixer).

The reaction mixture was diluted, and the analytes were quantified using HPLC with UV detection. The intermediates 7-keto-deoxycholic acid (7-keto-DCA) and 12-keto-chenodeoxycholic acid (12-keto-CDCA) were also detected (see Figure 2 for chemical structures).

**Table 3. Distribution of analytes in the samples containing NADH oxidases from different organisms for the regeneration of the cofactor NAD⁺.**

| source of NADH oxidase | distribution of analytes [%] | | | |
|---|---|---|---|---|
| | substrate: CA | 7-keto-DCA | 12-keto-CDCA | product: 7,12-diketo-LCA |
| ***Carnobacterium divergens* (SEQ ID No. 1)** | **0.0** | **0.1** | **0.0** | **99.9** |
| *Aerococcus urinaehominis* (NCBI Protein Database, *WP 067978261.1)* | 0.0 | 14.7 | 1.4 | 83.9 |
| *Weissella minor* (NCBI Protein Database, **WP_057787599.1**) | 28.6 | 54.9 | 3.4 | 13.0 |
| *Weissella jogaejeotgali* (NCBI Protein Database, WP_075269827.1) | 88.8 | 1.2 | 0.1 | 9.9 |
| *Enterococcus faecium* (NCBI Protein Database, WP_002334533.1) | 57.4 | 31.7 | 2.0 | 8.9 |

Table 3 shows that complete conversion the CA to 7,12-diketo-LCA can only be achieved when the cofactor regeneration is performed by the NADH oxidase from SEQ ID No. 1.

A similar enzymatic reaction (oxidation of 7-keto-DCA to 7,12-diketo-LCA) using the same 12α-hydroxysteroid dehydrogenase from *Eggerthella lenta* and commercially available NAD(P)H oxidase is described in the literature (Tonin et al., 2019). When using 10 mM (4,1 g/l) 7-keto-DCA as substrate, only 94% conversion could be achieved after 24 h. Much faster reaction rates (> 99% conversion after 4 h) were observed when the NAD(P)H oxidase was replaced by L-malate dehydrogenase as cofactor regeneration enzyme and oxaloacetate as sacrificial substrate. The authors concluded that the low activity of the employed NAD(P)H oxidase limits the reaction rate in their system.

In contrast, the system described in this example (combination of 7α- and 12α-hydroxysteroid dehydrogenases with the NADH oxidase according to SEQ ID No. 1) allows >99% conversion of 50 g/l cholic acid (122 mM) to 7,12-diketo-LCA in 20 h. This example shows that the high activity of the NADH oxidase from SEQ ID No. 1 is retained even in presence of high concentrations of bile acids.

### Example 7

### Oxidation of cholic acid (CA) to 12-keto-chenodeoxycholic acid (12-keto-CDCA) using NADH oxidase according to SEQ ID No. 1 and NADH oxidase from Streptococcus mutans as cofactor regeneration enzymes

A 20 g/l solution of cholic acid incubated in 50 mM potassium phosphate buffer (pH 8.0) containing 0.1 mM NAD⁺, 0.25 U of 12α-hydroxysteroid dehydrogenase from *Eggerthella lenta* (UniProt, C8WLK7) was prepared in two glass vials. To the first vial 0.74 µl (equivalent to 0.15 mg *E. coli* biomass) of a cell lysate containing NADH oxidase according to SEQ ID No. 1 (equivalent to 1 U) and to the second vial 1.1 µl (equivalent to 0.22 mg E. *coli* biomass) of a cell lysate containing NADH oxidase from *Streptococcus mutans* (equivalent to 0.5 U) are added. The reaction mixtures were incubated for 20 h at 25 °C and 800 rpm (Eppendorf Thermomixer).
300 µl of the reaction mixture was diluted with 600 µl of a mixture of acetonitrile/H₂O/50% H₃PO₄ (90/9/1) and incubated for 15 min at 55 °C and 800 rpm. After centrifugation (5 min at max. g) 200 µl of the supernatant was transferred to an HPLC vial. The analytes were quantified using HPLC with UV detection.

**Table 4. Distribution of analytes in the samples containing NADH oxidase according to SEQ ID No. 1 and NADH oxidase from Streptococcus mutans. The used cell lysates contained 20% (m/m) E. coli biomass.**

| source of NADH oxidase | biomass [mg] | distribution of analytes [%] | |
|---|---|---|---|
| | | substrate: CA | product: 12-keto-CDCA |
| *Carnobacterium divergens* **(SEQ ID No. 1)** | **0.15** | **18.5** | **81.5** |
| *Streptococcus mutans* | 0.22 | 49.2 | 50.8 |

Table 4 shows that a higher conversion can be achieved with a smaller amount of used *E. coli* biomass containing the NADH oxidase according to SEQ ID No. 1 when compared to the NADH oxidase from *Streptococcus mutans.*

The same enzymatic oxidation from cholic acid to 12-keto-CDCA was also performed by Tonin et al. The authors could achieve conversions of 78% after 24 h when using a purified 12α-HSDH from *Eggerthella lenta* (0.2 U), a commercial NAD(P)H oxidase (used enzyme units are not specified) and a substrate load of 8.2 g/l (20 mM) CA (Tonin et al., 2019).

In contrast, the system described in this example (12α-hydroxysteroid dehydrogenases and NADH oxidase according to SEQ ID No. 1) allows 81.5% conversion of 20 g/l cholic acid (49 mM) to 12-diketo-CDCA in 20 h. This example also illustrates the high activity of the NADH oxidase in presence of bile acids.

### Example 8

### a) Conversion of sugar alcohol allitol to corresponding ketose D-psicose (D-allulose) using NADH oxidase according to SEQ ID No. 1 as cofactor regeneration enzyme

125 µl of a 200 g/l allitol solution (50 g/l substrate load) was incubated in 100 mM triethanolamine buffer (pH 8.0) containing 0.1 mM NAD⁺. 50 µl of cell lysate comprising xylitol dehydrogenase from *Galactocandida mastotermitis* (*Candida* sp. HA167) (UniProt, O74230) and 7.4 U of NADH oxidase according to SEQ ID No. 1 (applied as cell lysate) were added, and the resulting mixture was incubated at 30 °C and 800 rpm (Eppendorf Thermomixer) .

After 20 h of incubation 100 µl of the reaction mixture was mixed with 200 µl MeOH and incubated at 60 °C and 1200 rpm for 15 min (Eppendorf Thermomixer). 700 µl of deionized water was added and the mixture was centrifuged for 5 min at max. g. 200 µl of the supernatant was transferred to an HPLC vial and the analytes were quantified using HPLC with refractive index detection.

The HPLC data show that allitol was completely oxidized to D-psicose (see Figure 2 for chemical structures). *b) Conversion of sugar alcohol allitol to corresponding ketose D-psicose (D-allulose) using NAD(P)H oxidase according to SEQ ID No. 17 as cofactor regeneration enzyme*

125 µl of a 200 g/l allitol solution (50 g/l substrate load) was incubated in 100 mM triethanolamine buffer (pH 8.0) containing 0.05 mM NADP⁺. 25 µl of cell lysate containing a short chain dehydrogenase/reductase from *Gluconobacter frateurii* (NCBI Protein Database, WP_063903495.1) and 10 U of NAD(P)H oxidase according to SEQ ID No. 17 (applied as cell lysate) were added, and the resulting mixture was incubated at 24 °C and 800 rpm (Eppendorf Thermomixer).

After 20 h of incubation 100 µl of the reaction mixture was mixed with 200 µl MeOH and incubated at 60 °C and 1200 rpm for 15 min (Eppendorf Thermomixer). 700 µl of deionized water was added and the mixture was centrifuged for 5 min at max. g. 200 µl of the supernatant was transferred to an HPLC vial and the analytes were quantified using HPLC with refractive index detection.

The HPLC data show that 69% of the allitol was oxidized to D-psicose (see Figure 2 for chemical structures).

This example demonstrates that both the NAD(P)H oxidases according to SEQ ID No. 1 and SEQ ID No. 17 permit the efficient cofactor regeneration (NAD⁺ or NAD(P)⁺, respectively) to oxidize the sugar alcohol allitol to the corresponding ketose D-psicose, a rare sugar used as low-calorie sweetener.

### Example 9

### Kinetic characterization of NADH oxidase WT and mutants (Seq ID No. 1, 18, 19, 20, 21)

Activity measurements with enzyme preparations were performed as follows:
A defined volume (10 µl) of enzyme dilution was mixed in a cuvette of a total volume of one mL consisting of 0.2 mM cofactor (NADH and NADPH, respectively) and 100 mM potassium pyrophosphate pH 7.0. The change of absorbance (ΔA) measured at 340 nm is a measure of enzyme activity according to Lambert-Beer's law (ΔA = ε-Δc·d; where ε is 6.22 l/(mmol·cm) for NAD(P)H; Δc is the change in concentration and d is the optical path length of the cuvette (1 cm). One enzyme unit U corresponds to the amount of enzyme that catalyzes the conversion of one µmol substrate per minute under defined conditions. The activity given in Table 5 below is expressed as U/g biomass (wet weight).

**Table 5. Activity parameters for the NAD(P)H oxidases according to SEQ ID No. 1 and 16-19.**

| **Seq ID No.** | | **NADH (U/g)** | | **NADPH (U/g)** | | **NADH (%)** | | **NADPH (%)** | |
|---|---|---|---|---|---|---|---|---|---|
| | **1** | | 3055 | | 9 | | 99.7 | | 0.3 |
| | **16** | | 1228 | | 3174 | | 28 | | 72 |
| | **17** | | 113 | | 1625 | | 6 | | 94 |
| | **18** | | 1579 | | 2646 | | 37 | | 63 |
| | **19** | | 315 | | 3572 | | 8 | | 92 |

### LITERATURE

Chenault, H. K., Simon, E. S., & Whitesides, G. M. (1988). Cofactor regeneration for enzyme-catalysed synthesis. Biotechnology & Genetic Engineering Reviews, 6, 221-270. https://doi.org/10.1080/02648725.1988.10647849
Wang, X., Saba, T., Yiu, H. H. P., Howe, R. F., Anderson, J. A., & Shi, J. (2017). Cofactor NAD(P)H Regeneration Inspired by Heterogeneous Pathways. Chem, 2(5), 621-654. https://doi.org/10.1016/j.chempr.2017.04.009
Geueke, B., Riebel, B., & Hummel, W. (2003). NADH oxidase from Lactobacillus brevis: a new catalyst for the regeneration of NAD. Enzyme and Microbial Technology, 32(2), 205-211. https://doi.org/10.1016/S0141-0229(02) 0 0290-9
Ward, D. E., Donnelly, C. J., Mullendore, M. E., van der Cost, J., de Vos, W. M., & Crane, E. J., 3rd (2001). The NADH oxidase from Pyrococcus furiosus. Implications for the protection of anaerobic hyperthermophiles against oxidative stress. European Journal of Biochemistry, 268(22), 5816-5823. https://doi.org/10.1046/j.0014-2956.2001.02526.x
Riebel, B. R., Gibbs, P. R., Wellborn, W. B., & Bommarius, A. S. (2002). Cofactor Regeneration of NAD+ from NADH: Novel Water-Forming NADH Oxidases. Advanced Synthesis and Catalysis, 344(10), 1156-1168. https://doi.org/10.1002/1615-4169(200212)344:10<1156::AID-ADSC1156>3.0.CO;2-%23
Higuchi, M., Shimada, M., Yamamoto, Y., Hayashi, T., Koga, T., & Kamio, Y. (1993). Identification of two distinct NADH oxidases corresponding to H2O2-forming oxidase and H2O-forming oxidase induced in Streptococcus mutans. Journal of General Microbiology, 139(10), 2343-2351. https://doi.org/10.1099/00221287-139-10-2343
Matsumoto, J., Higuchi, M., Shimada, M., Yamamoto, Y., & Kamio, Y. (1996). Molecular cloning and sequence analysis of the gene encoding the H2O-forming NADH oxidase from Streptococcus mutans. Bioscience, Biotechnology, and Biochemistry, 60(1), 39-43. https://doi.org/10.1271/bbb.60.39
Higuchi, M., Yamamoto, Y., Poole, L. B., Shimada, M., Sato, Y., Takahashi, N., & Kamio, Y. (1999). Functions of two types of NADH oxidases in energy metabolism and oxidative stress of Streptococcus mutans. Journal of Bacteriology, 181(19), 5940-5947. https://doi.org/10.1128/JB.181.19.5940-5947.1999
Riebel, B. R., Gibbs, P. R., Wellborn, W. B., & Bommarius, A. S. (2003) . Cofactor Regeneration of both NAD+ from NADH and NADP+ from NADPH:NADH Oxidase from Lactobacillus sanfranciscensis. Advanced Synthesis and Catalysis, 345(6-7), 707-712. https://doi.org/10.1002/adsc.200303039
Klibanov, A. M. (1997). Why are enzymes less active in organic solvents than in water? Trends in Biotechnology, 15(3), 97-101. https://doi.org/10.1016/S0167-7799(97)01013-5
Tonin, F., & Arends, I. W. C. E. (2018). Latest development in the synthesis of ursodeoxycholic acid (UDCA): a critical review. Beilstein Journal of Organic Chemistry, 14, 470-483. https://doi.org/10.3762/bjoc.14.33
Tonin, F., Alvarenga, N., Ye, J. Z., Arends, I. W. C. E., & Hanefeld, U. (2019). Clean Enzymatic Oxidation of 12α-Hydroxys-teroids to 12-Oxo-Derivatives Catalyzed by Hydroxysteroid Dehydrogenase Advances Synthesis & Catalysis, 361(11), 2448-2455. https://doi.org/10.1002/adsc.201900144
Cremers, C. M., Knoefler, D., Vitvitsky, V., Banerjee, R., & Jakob, U. (2014). Bile salts act as effective protein-unfolding agents and instigators of disulfide stress in vivo. Proceedings of the National Academy of Sciences of the United States of America, 111(16), E1610-E1619. https://doi.org/10.1073/pnas.1401941111
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Jiang, S., Xiao, W., Zhu, X., Yang, P., Zheng, Z., Lu, S., Jiang, S., Zhang, G., & Liu, J. (2020). Review on D Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.

## Claims

1. A protein having NADH and/or NADPH oxidase activity comprising an amino acid sequence selected from the group consisting of
i) an amino acid sequence having at least 80% sequence identity with SEQ ID No. 1,
ii) an amino acid sequence encoded by a nucleic acid sequence having an identity to SEQ ID No. 2 of at least 80%, and
iii) an amino acid sequence encoded by a nucleic acid which binds under stringent conditions to a nucleic acid molecule complementary to the nucleic acid sequence SEQ ID No. 2.

2. The protein according to claim 1, wherein said protein comprises a polypeptide consisting of the amino acid sequence GX₁GX₂X₃X₄, wherein X₁ is a non-polar amino acid residue, X₂ is a non-polar amino acid residue, X₃ is a non-polar amino acid residue and X₄ is a non-polar amino acid residue.

3. The protein according to claim 2, wherein X₁ is glycine, and/or X₂ is tyrosine, and/or X₃ is isoleucine, and/or X₄ is glycine or alanine.

4. The protein according to any one of claims 1 to 3, wherein said protein comprises a polypeptide consisting of the amino acid sequence GX₁GX₂X₃X₄X₅X₆X₇X₈X₉X₁₀X₁₁X₁₂, wherein X₅ is a peptide consisting of 6 to 12, preferably 8 to 10, more preferably 10, amino acid residues, X₆ is a non-polar or a polar amino acid residue, X₇ is a peptide consisting of 4 to 8, preferably 4 to 6, more preferably 6, amino acid residues, X₈ is an acidic amino acid residue or a non-polar amino acid residue, X₉ is a non-polar amino acid residue or a basic amino acid residue, X₁₀ is a non-polar amino acid residue or a polar amino acid residue, X₁₁ is a peptide consisting of 2 to 6, preferably 2 to 4, more preferably 4, amino acid residues, and X₁₂ is a polar amino acid residue or a basic amino acid residue.

5. The protein according to claim 4, wherein X₅ is a peptide consisting of amino acid sequence IELVEAFAES (SEQ ID No. 9) and/or X₇ is a peptide consisting of amino acid sequence KQVTLV (SEQ ID No. 10) and/or X₁₁ is a peptide consisting of amino acid sequence DRIL (SEQ ID No. 11).

6. The protein according to claim 4 or 5, wherein X₆ is glycine or asparagine and/or X₈ is aspartic acid or alanine and/or X₉ is glycine or arginine and/or X₁₀ is leucine or serine and/or X₁₂ is asparagine or arginine.

7. The protein according to any one of claims 4 to 6, wherein X₄ is non-polar and X₆ is a polar amino acid residue, preferably glycine and asparagine, respectively, X₈ is a non-polar amino acid residue, preferably alanine, X₁₀ is a polar amino acid residue, preferably serine, and X₉ and X₁₂ are a basic amino acid residue, preferably arginine.

8. The protein according to any one of claims 4 to 6, wherein X₄ is a non-polar amino acid residue, preferably alanine, X₆ is a non-polar or a polar amino acid residue, preferably glycine and asparagine, respectively, X₈ is a non-polar amino acid residue, preferably alanine, X₁₀ is a polar amino acid residue, preferably serine, and X₉ and X₁₂ are a basic amino acid residue, preferably arginine.

9. The protein according to any one of claims 4 to 6, wherein X₄ and X₆ are non-polar amino acid residue, preferably glycine, X₈ is an acidic amino acid residue, preferably aspartic acid, X₉ is a non-polar amino acid residue, preferably glycine, X₁₀ is a non-polar amino acid residue, preferably leucine, and X₁₂ is a polar amino acid residue, preferably asparagine.

10. A nucleic acid molecule encoding a protein according to any one of claims 1 to 9.

11. Vector comprising a nucleic acid molecule according to claim 10.

12. Host cell comprising a nucleic acid molecule according to claim 10 or a vector according to claim 11.

13. Method for oxidizing NADH to NAD⁺ and/or NADPH to NADP⁺ comprising the step of incubating a protein according to any one of claims 1 to 9 or a host cell according to claim 12 or a lysate or homogenate of said host cell with NADH and/or NADPH.

14. Method for the enzymatic oxidation of a compound, where NADH is formed from NAD⁺ and/or NADPH is formed from NADP⁺, comprising the step of oxidizing NADH to NAD⁺ and/or NADPH to NADP⁺ by adding a protein according to any one of claims 1 to 9 or a host cell according to claim 12 or a lysate or homogenate of said host cell.

15. Method according to claim 13 or 14, wherein the oxidation reaction is performed in an aqueous solution comprising at least one organic co-solvent.
